# EUROPÄISCHE PATENTSCHRIFT

(11) **EP 4 001 255 B1**
(45) Veröffentlichungstag und Bekanntmachung des Hinweises auf die Patenterteilung: **27.05.2026**
(21) Anmeldenummer: 20209161.7
(22) Anmeldetag: 23.11.2020
(51) Int. Cl.: C07C 67/38, C07C 69/14

(54) **RU-KATALYSIERTE DOMINO HYDROFORMYLIERUNG/HYDRIERUNG/VERESTERUNG UNTER EINSATZ VON PHOSPHIN-LIGANDEN**
RU-CATALYZED DOMINO HYDROFORMYLATION/HYDROGENATION/ESTERIFICATION USING PHOSPHINE LIGANDS
HYDROFORMYLATION/HYDROGÉNATION/ESTERIFICATION DOMINO CATALYSÉE RU À L'AIDE DES LIGANDS DE PHOSPHINE

(43) Veröffentlichungstag der Anmeldung: 25.05.2022
(73) Patentinhaber: Evonik Oxeno GmbH & Co. KG, 45772 Marl (DE)
(72) Erfinder: KUCMIERCZYK, Peter, 44628 Herne (DE); FRANKE, Robert, 45772 Marl (DE); BELLER, Matthias, 18211 Ostseebad Nienhagen (DE); DÜHREN, Ricarda, 18069 Rostock (DE); SCHNEIDER, Carolin, 40789 Monheim am Rhein (DE); JACKSTELL, Ralf, 18106 Rostock (DE)
(74) Vertreter: Evonik Patent Association

(56) Entgegenhaltungen:
- EP-A1- 2 975 019
- EP-A1- 3 272 732
- EP-A1- 3 441 383
- US-A- 3 168 553

## Beschreibung

Die vorliegende Erfindung betrifft eine Ru-katalysierte Domino Hydroformylierung/Hydrierung/Veresterung unter Einsatz von Phosphin-Liganden.

In EP 0 329 252 B1 wird ein Verfahren zur Herstellung von Carbonsäuren oder Estern beschreiben. Das Verfahren wird hierbei durch den Einsatz einer Rutheniumverbindung in Kombination mit einer sauren Verbindung katalysiert. Die saure Verbindung umfasst hierbei eines der folgenden Elemente: Phosphor, Antimon, Arsen, Molybdän, Wolfram. In US 3 168 553 A wird ein Alkoxycarbonylierungsverfahren zur Herstellung von Ethylpropionat beschrieben.

In I. Fleischer, K. M. Dyballa, R. Jennerjahn, R. Jackstell, R. Franke, A. Spannenberg, M. Beller, "From Olefins to Alcohols: Efficient and Regioselective Ruthenium-Catalyzed Domino Hydroformylation/Reduction Sequence", Angew. Chem. Int. Ed. 2013, 52, 2949 - 2953 wir ein Verfahren zur Hydroformylierung und anschließender Reduktion beschrieben. Das eingesetzte Olefin wird hierbei zum Aldehyd umgesetzt und abschließend zum Alkohol reduziert.

Die technische Aufgabe, welche der vorliegenden Erfindung zugrunde lag, ist die Bereitstellung eines Verfahrens, welches ausgehend von einem Olefin direkt zu einem Ester führt, ohne dass Zwischenstufen isoliert werden. Wobei das ursprüngliche Olefin den Alkoholteil des Esters ausbilden soll, d.h. über den Sauerstoff an das Kohlenstoffatom der C=O-Gruppe gebunden ist. Des Weiteren soll der Alkoholteil des Esters über einen Kohlenstoff mehr verfügen, als das eingesetzte Olefin.

Diese Aufgabe wird gelöst durch ein Verfahren gemäß Anspruch 1.

Verfahren umfassend die Verfahrensschritte:
a) Vorlegen einer ethylenisch ungesättigten Verbindung;
b) Zugabe eines Liganden gemäß der Formel (I): 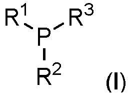 wobei
   R¹, R², R³ ausgewählt sind aus: -(C₁-C₁₂)-Alkyl, -(C₆-C₁₂)-Cycloalkyl, -(C₆-C₂₀)-Aryl, wobei der -(C₆-C₁₂)-Cycloalkyl-Rest und der -(C₆-C₂₀)-Aryl-Rest Substituenten aufweisen können, welche ausgewählt sind aus: -(C₁-C₁₂)-Alkyl, -O-(C₁-C₁₂)-Alkyl, - SO₃Na;
   und einer Verbindung, welche Ru umfasst;
c) Zugabe einer Säure (II), welche die Formel (IIa) oder (IIb) aufweist: 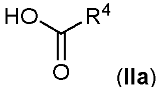 wobei R⁴ für -(C₁-C₁₈)-Alkyl steht; 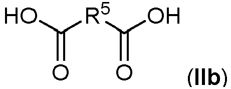 wobei R⁵ für -(C₁-C₁₈)-Alkyl steht;
d1) Zuführen von CO; d3) Zugabe von H₂O, wobei H₂O in einer Menge zugegeben wird, so dass das molare Verhältnis von H₂O zu der ethylenisch ungesättigten Verbindung im Bereich von 1:1 bis 10:1 liegt;
e) Erwärmen des Reaktionsgemisches aus a) bis d), wobei die ethylenisch ungesättigte Verbindung direkt zu einem Ester beziehungsweise Diester der Säure (II) umgesetzt wird, ohne dass Zwischenstufen isoliert werden.

Hierbei umfasst d) die Verfahrensschritte d1) und d3), und gegebenenfalls die weiteren Verfahrensschritte d2) und und d4).

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff (C₁-C₁₂)-Alkyl umfasst geradkettige und verzweigte Alkylgruppen mit 1 bis 12 Kohlenstoffatomen. Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₁₂)-Cycloalkyl umfasst mono-, bi- oder tricyclische Kohlenwasserstoffgruppen.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein. Vorzugsweise weist die ethylenisch ungesättigten Verbindungen eine Kohlenstoff-Kohlenstoff-Doppelbindungen auf.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform ist die ethylenisch ungesättigte Verbindung ausgewählt aus: Ethen, Propen, 1-Buten, cis und/oder trans-2-Buten, iso-Buten, 1,3-Butadien, 1-Penten, cis-und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Ausführungsform ist R¹ ausgewählt aus: -(C₁-C₁₂)-Alkyl, -(C₆-C₂₀)-Aryl, -(C₅-C₂₀)-Heteroaryl.

In einer Ausführungsform sind R¹, R², R³ ausgewählt aus: -(C₆-C₁₂)-Cycloalkyl, -(C₆-C₂₀)-Aryl.

In einer Ausführungsform stehen R¹, R², R³ für -(C₆-C₁₂)-Cycloalkyl.

In einer Ausführungsform stehen R¹, R², R³ für -Cy.

In einer Ausführungsform stehen R¹, R², R³ für -(C₆-C₂₀)-Aryl.

In einer Ausführungsform stehen R¹, R², R³ für -Ph.

In einer Ausführungsform stehen R¹, R², R³ für den gleichen Rest.

In einer Ausführungsform ist die Verbindung, welche Ru umfasst, ausgewählt aus: Ru₃(CO)₁₂, RuCl₃*H₂O, Ru(Cl)₂(DMSO)₄, Ru(acac)₃.

In einer Ausführungsform ist die Verbindung, welche Ru umfasst, Ru₃(CO)₁₂.

In einer Ausführungsform steht R⁴ für -(C₁-C₁₂)-Alkyl.

In einer Ausführungsform steht R⁴ für -(C₁-C₈)-Alkyl.

In einer Ausführungsform steht R⁵ für -(C₁-C₁₂)-Alkyl.

In einer Ausführungsform steht R⁵ für -(C₁-C₈)-Alkyl.

In einer Ausführungsform umfasst das Verfahren den zusätzlichen Verfahrensschritt d2): d2) Zuführen von H₂.

In einer Ausführungsform liegt der H₂-Druck im Bereich von 1 MPa (10 bar) bis 6 MPa (60 bar).

In einer Ausführungsform umfasst das Verfahren den zusätzlichen Verfahrensschritt d4): d4) Zugabe von para-Toluolsulfonsäure.

In einer Ausführungsform wird die Säure (II) in Verfahrensschritt c) in einer Menge zugegeben, so dass das molare Verhältnis von Säure zu der ethylenisch ungesättigten Verbindung im Bereich von 2:1 bis 10:1 liegt.

In einer Ausführungsform weist die Säure (II) die Formel (IIa) auf.

In einer Ausführungsform weist die Säure (II) die Formel (IIb) auf.

In einer Ausführungsform ist der Ligand in Verfahrensschritt b) ausgewählt aus:

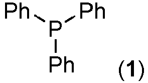

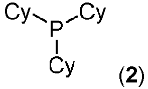

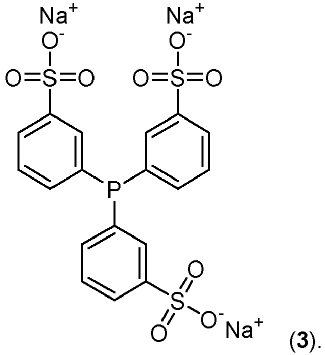

In einer Ausführungsform wird die Reaktionsmischung im Verfahrensschritt e) auf eine Temperatur zwischen 50 °C und 180 °C, bevorzugt zwischen 80 °C und 160 °C, besonders bevorzugt zwischen 100 °C und 150 °C erwärmt, um die ethylenisch ungesättigte Verbindung zum Ester umzusetzen.

Im Folgenden wird die Erfindung anhand von Ausführungsbeispielen näher beschrieben.

### Allgemeine Arbeitsvorschriften

Alle Arbeiten mit luft- und feuchtigkeitsempfindlichen Stoffen wurden in einer Argonatmosphäre und mit ausgeheizten Glasgeräten unter Verwendung der Schlenk-Technik durchgeführt. Die Chemikalien wurden von kommerziellen Herstellern bezogen und so verwendet wie sie geliefert wurde, wenn die Reinheit mindestens 98% betrug. Sauerstofffreie und trockene Lösungsmittel wurden mittels Destillation unter Argon vorbereitet. Synthesegas (CO:99,997%, H₂/CO:1:1+/-1%) wurde von der Firma Linde bezogen.

Die Produkte wurden mittels ¹H-NMR- und ¹³C-NMR-Spektroskopie analysiert. Die NMR-Spektren wurden auf Geräten des Typs Bruker AV 400 (400 MHz), Bruker AV 300 (300 MHz), oder Fourier 300 (MHz) aufgenommen. Chemische Verschiebungen δ (ppm) werden relativ zum verwendeten Lösungsmittel angegeben: Referenzen für CDCl₃ waren 7.26 ppm (¹H-NMR) und 77.16 (¹³C-NMR). ¹³C-NMR-Spekren wurden mit einer breitbandentkoppelnden Methode aufgenommen.

GC-Analysen wurden auf einem 7890A GC-System der Firma Agilent Technologies mit einer 30 m HP-5 Säule durchgeführt. Als Trägergas wurde Argon verwendet. Die Produkte wurden mittels GC oder GC-MS analysiert oder mittels Säulenchromatographie (Silika, EtOAc/Heptan) isoliert. Die GC-Ausbeuten wurden über interne Kalibrierung berechnet. Als interner Standard wurde Hexadecan verwendet.

### Durchführung der katalytischen Experimente

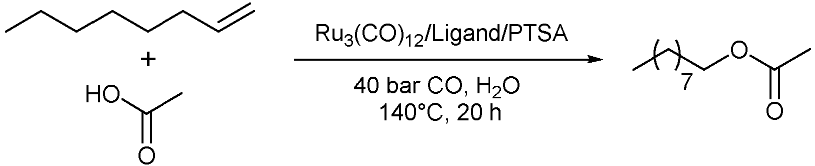

Die katalytischen Experimente wurden in 4 mL Glasvials mit Schraubverschlusskappen und einem PTFE-Septum durchgeführt. Die Vials wurden mit ofengetrockneten Magnetrührern bestückt und über eine Nadel die Verbindung zur Gasatmosphäre hergestellt. Der Reaktionsansatz betrug 2 mL. Die Vials wurden in einem 300 mL Parr4560 Autoklav platziert und über einem Magnetrührwerk gerührt. Im ersten Schritt werden Ru₃(CO)₁₂ (5mol%), Ligand (5.5mol%) und PTSA*H₂O (20.6 mol%) in das Vial eingewogen. Das Vial wird mit einer Schraubverschlusskappe, die mit einem Septum versehen ist, verschlossen und über eine Kanüle mit der Argonatmosphäre verbunden. Das Vial wurde dreimal sekuriert und mit Argon gespült. Essigsäure (1.17 mL), H₂O (0.35 mL) und 1-Octen (3 mmol) wurde mittels Hamiltonspritze injiziert. Unter Argonatmosphäre wurde das Vial in den Autolaven überführt. Der Autoklav wird fest verschlossen und zunächst bei Raumtemperatur dreimal mit 10 bar CO gespült. Danach werden 40 bar CO aufgepresst, der Autoklav in einem Aluminiumblock auf einem Magnetrührwerk platziert und für 20 h auf 140 °C geheizt. Nach 20 h wurde der Autoklav auf Raumtemperatur heruntergekühlt und der Druck vorsichtig abgelassen. Als interner Standard wurden 100 µL Hexadecan in die Reaktionslösung gegeben. Die Ausbeute wurden mittels GC-Analyse bestimmt.

Die Reaktion wurde unter analogen Bedingungen für die Liganden **(1)** bis **(3),** sowie für den Vergleichsliganden **(4)** durchgeführt. Da es sich bei dem Vergleichsliganden **(4)** um einen bidentaten Liganden handelt wurde anstelle der 5,5 mol% nur 2,75 mol% eingesetzt. Die Um ein konstantes molares Verhältnis von PTSA*H₂O: L zu erhalten (3,75:1) wurde die Menge dann auf 10,3 mol% reduziert.

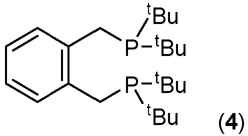

### Reaktionsbedingungen

1-Octene: 3 mmol
Ru₃(CO)₁₂: 5 mol% Ru
Ligand **(1)** bis **(3):** 5,5 mol% bezogen auf 1-Octen
Ligand **(4):** 2,75 mol% bezogen auf 1-Octen
PTSA*H₂O unter Verwendung von **(1)** bis (3): 20,6 mol%
PTSA*H₂O unter Verwendung von **(4):** 10,3 mol%
H₂O : 1-Octene = 6,5 : 1 (Molverhältnis)
HOAc : 1- Octene = 6,75 : 1 (Molverhältnis)
CO-Druck: 40 bar
Temperatur: 140 °C
Reaktionszeit: 20 h

### Versuchsergebnisse

| Ligand | Ausbeute Ester [%] |
|---|---|
| (**1**)* | 24 |
| (**2**)* | 25 |
| (**3**)* | 23 |
| (**4**) | 10 |

| | |
|---|---|
| * erfindungsgemäßes Verfahren | |

## Patentansprüche

1. Verfahren umfassend die Verfahrensschritte:
a) Vorlegen einer ethylenisch ungesättigten Verbindung;
b) Zugabe eines Liganden gemäß der Formel (**I**): 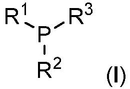 wobei
R¹, R², R³ ausgewählt sind aus: -(C₁-C₁₂)-Alkyl, -(C₆-C₁₂)-Cycloalkyl, -(C₆-C₂₀)-Aryl, wobei der -(C₆-C₁₂)-Cycloalkyl-Rest und der -(C₆-C₂₀)-Aryl-Rest Substituenten aufweisen können, welche ausgewählt sind aus: -(C₁-C₁₂)-Alkyl, -O-(C₁-C₁₂)-Alkyl, - SO₃Na;
und einer Verbindung, welche Ru umfasst;
c) Zugabe einer Säure (**II**), welche die Formel (**IIa**) oder (**IIb**) aufweist: 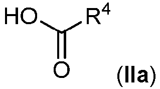 wobei R⁴ für -(C₁-C₁₈)-Alkyl steht; 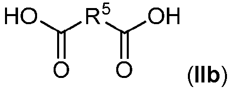 wobei R⁵ für -(C₁-C₁₈)-Alkyl steht;
d1) Zuführen von CO;
d3) Zugabe von H₂O, wobei H₂O in einer Menge zugegeben wird, so dass das molare Verhältnis von H₂O zu der ethylenisch ungesättigten Verbindung im Bereich von 1:1 bis 10:1 liegt;
e) Erwärmen des Reaktionsgemisches aus a) bis d), wobei die ethylenisch ungesättigte Verbindung direkt zu einem Ester beziehungsweise Diester der Säure (II) umgesetzt wird, ohne dass Zwischenstufen isoliert werden.

2. Verfahren nach Anspruch 1,
wobei die ethylenisch ungesättigte Verbindung ausgewählt ist aus: Ethen, Propen, 1-Buten, cis und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

3. Verfahren nach einem der Ansprüche 1 oder 2,
wobei R¹, R², R³ ausgewählt sind aus: -(C₆-C₁₂)-Cycloalkyl, -(C₆-C₂₀)-Aryl.

4. Verfahren nach einem der Ansprüche 1 bis 3,
wobei R¹, R², R³ für den gleichen Rest stehen.

5. Verfahren nach einem der Ansprüche 1 bis 4,
wobei die Verbindung, welche Ru umfasst, ausgewählt ist aus: Ru₃(CO)₁₂, RuCl₃*H₂O, Ru(Cl)₂(DMSO)₄, Ru(acac)₃.

6. Verfahren nach einem der Ansprüche 1 bis 5,
wobei R⁴ für -(C₁-C₁₂)-Alkyl steht.

7. Verfahren nach einem der Ansprüche 1 bis 6,
wobei R⁵ für -(C₁-C₁₂)-Alkyl steht.

8. Verfahren nach einem der Ansprüche 1 bis 7,
umfassend den zusätzlichen Verfahrensschritt d2):
d2) Zuführen von H₂.

9. Verfahren nach Anspruch 8,
wobei der H₂-Druck im Bereich von 1 MPa (10 bar) bis 6 MPa (60 bar) liegt.

10. Verfahren nach einem der Ansprüche 1 bis 9,
umfassend den zusätzlichen Verfahrensschritt d4):
d4) Zugabe von para-Toluolsulfonsäure.

11. Verfahren nach einem der Ansprüche 1 bis 10,
wobei die Säure (**II**) in Verfahrensschritt c) in einer Menge zugegeben wird, so dass das molare Verhältnis von Säure zu der ethylenisch ungesättigten Verbindung im Bereich von 2:1 bis 10:1 liegt.

12. Verfahren nach einem der Ansprüche 1 bis 11,
wobei die Säure (**II**) die Formel (**IIa**) aufweist.

13. Verfahren nach einem der Ansprüche 1 bis 12,
wobei der Ligand in Verfahrensschritt b) ausgewählt ist aus:

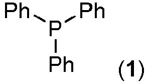

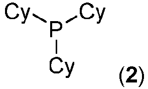

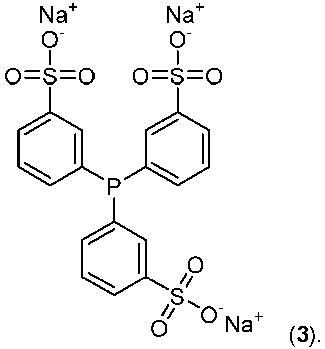

## Claims

1. Process comprising the process steps of:
a) initially charging an ethylenically unsaturated compound;
b) adding a ligand of formula (I): 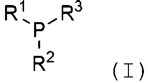 wherein
R¹, R², R³ are selected from: -(C₁-C₁₂)-alkyl, -(C₆-C₁₂)-cycloalkyl, -(C₆-C₂₀)-aryl,
wherein the -(C₆-C₁₂)-cycloalkyl radical and the -(C₆-C₂₀)-aryl radical may have substituents which are selected from: -(C₁-C₁₂)-alkyl, -O-(C₁-C₁₂)-alkyl, -SO₃Na;
and a compound comprising Ru;
c) adding an acid (II) having the formula (IIa) or (IIb) : 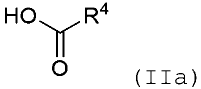 wherein R⁴ is -(C₁-C₁₈)-alkyl; 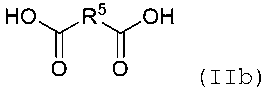 wherein R⁵ is -(C₁-C₁₈)-alkyl;
d1) supplying CO;
d3) adding H₂O, wherein H₂O is added in an amount such that the molar ratio of H₂O to the ethylenically unsaturated compound is in the range from 1:1 to 10:1;
e) heating the reaction mixture from a) to d) to convert the ethylenically unsaturated compound directly into an ester or diester of acid (II) without isolation of intermediates.

2. Process according to Claim 1,
wherein the ethylenically unsaturated compound is selected from: ethene, propene, 1-butene, cis- and/or trans-2-butene, isobutene, 1,3-butadiene, 1-pentene, cis- and/or trans-2-pentene, 2-methyl-1-butene, 3-methyl-1-butene, 2-methyl-2-butene, hexene, tetramethylethylene, heptene, 1-octene, 2-octene, di-n-butene, or mixtures thereof.

3. Process according to either of Claims 1 or 2,
wherein R¹, R², R³ are selected from: -(C₆-C₁₂)-cycloalkyl, -(C₆-C₂₀)-aryl.

4. Process according to any of Claims 1 to 3,
wherein R¹, R², R³ are the same radical.

5. Process according to any of Claims 1 to 4,
wherein the compound comprising Ru is selected from: Ru₃(CO)₁₂, RuCl₃*H₂O, Ru(Cl)₂(DMSO)₄, Ru(acac)₃.

6. Process according to any of Claims 1 to 5,
wherein R⁴ is -(C₁-C₁₂)-alkyl.

7. Process according to any of Claims 1 to 6,
wherein R⁵ is -(C₁-C₁₂)-alkyl.

8. Process according to any of Claims 1 to 7, comprising the additional process step d2):
d2) supplying H₂.

9. Process according to Claim 8,
wherein the H₂ pressure is in the range from 1 MPa (10 bar) to 6 MPa (60 bar).

10. Process according to any of Claims 1 to 9, comprising the additional process step d4):
d4) adding para-toluenesulfonic acid.

11. Process according to any of Claims 1 to 10,
wherein the acid (II) is added in process step c) in an amount such that the molar ratio of acid to the ethylenically unsaturated compound is in the range from 2:1 to 10:1.

12. Process according to any of Claims 1 to 11,
wherein the acid (II) has the formula (IIa).

13. Process according to any of Claims 1 to 12,
wherein the ligand in process step b) is selected from:

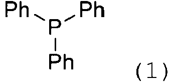

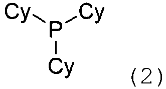

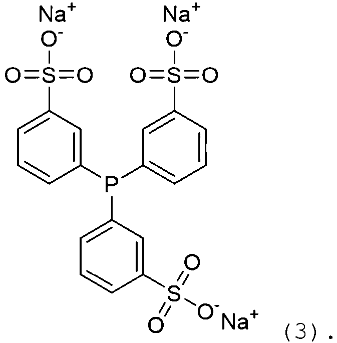

## Revendications

1. Procédé comprenant les étapes de procédé :
a) présentation d'un composé éthyléniquement insaturé ;
b) ajout d'un ligand selon la formule (I) : 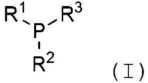 où
R¹, R², R³ sont choisis parmi : - (C₁-C₁₂)-alkyle, -(C₆-C₁₂)-cycloalkyle, -(C₆-C₂₀)-aryle,
où le radical -(C₆-C₁₂)-cycloalkyle et le radical -(C₆-C₂₀) -aryle peuvent présenter des substituants choisis parmi : -(C₁-C₁₂)-alkyle, -O-(C₁-C₁₂)-alkyle, -SO₃Na ;
et un composé, qui comprend du Ru ;
c) ajout d'un acide (II) qui présente la formule (IIa) ou (IIb) : 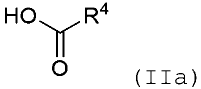 R⁴ représentant -(C₁-C₁₈)-alkyle ; 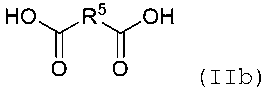 R⁵ représentant -(C₁-C₁₈)-alkyle ;
d1) fourniture de CO ;
d3) ajout d'H₂O, H₂O étant ajouté en une quantité telle que le rapport molaire de H₂O au composé éthyléniquement insaturé se situe dans la plage de 1:1 à 10:1 ;
e) chauffage du mélange réactionnel de a) à d), le composé éthyléniquement insaturé est converti directement en un ester ou diester de l'acide (II) sans isoler les intermédiaires.

2. Procédé selon la revendication 1,
dans lequel le composé éthyléniquement insaturé est choisi parmi : l'éthylène, le propène, le 1-butène, le cis- et/ou trans-2-butène, l'isobutène, le 1,3-butadiène, le 1-pentène, le cis- et/ou trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

3. Procédé selon l'une des revendications 1 ou 2,
R¹, R², R³ étant choisis parmi : -(C₆-C₁₂)-cycloalkyle, - (C₆-C₂₀)-aryle.

4. Procédé selon l'une des revendications 1 à 3,
où R¹, R², R³ représente le même radical.

5. Procédé selon l'une des revendications 1 à 4,
le composant qui comprend du Ru étant choisie parmi : Ru₃(CO)₁₂, RuCl₃*H₂O, Ru(Cl)₂(DMSO)₄, Ru(acac)₃.

6. Procédé selon l'une des revendications 1 à 5,
R⁴ représentant -(C₁-C₁₂)-alkyle.

7. Procédé selon l'une des revendications 1 à 6,
R⁵ représentant -(C₁-C₁₂)-alkyle.

8. Procédé selon l'une des revendications 1 à 7, comprenant l'étape de procédé supplémentaire d2) :
d2) fourniture de H₂.

9. Procédé selon la revendication 8,
dans lequel la pression de H₂ se situe dans la plage de 1 MPa (10 bars) à 6 MPa (60 bars).

10. Procédé selon l'une des revendications 1 à 9, comprenant l'étape de procédé supplémentaire d4) :
d4) ajout d'acide para-toluènesulfonique.

11. Procédé selon l'une des revendications 1 à 10,
dans lequel l'acide (II) est ajouté dans l'étape de procédé c) en une quantité telle que le rapport molaire de l'acide au composé éthyléniquement insaturé se situe dans la plage de 2:1 à 10:1.

12. Procédé selon l'une des revendications 1 à 11,
dans lequel l'acide (II) a la formule (IIa).

13. Procédé selon l'une des revendications 1 à 12,
dans lequel le ligand dans l'étape de procédé b) est choisi parmi :
